# EUROPÄISCHE PATENTANMELDUNG

(11) **EP 4 357 019 A1**
(43) Veröffentlichungstag der Anmeldung: **24.04.2024**
(21) Anmeldenummer: 22202894.6
(22) Anmeldetag: 21.10.2022
(51) Int. Cl.: B01J 27/198, B01J 35/00, B01J 35/02, B01J 37/00, C07C 51/215

(54) **VPO-KATALYSATOR MIT VERBESSERTER SELEKTIVITÄT UND STABILITÄT SOWIE VERFAHREN ZU DESSEN HERSTELLUNG**

(71) Anmelder: Clariant International Ltd, 4132 Muttenz (CH)
(72) Erfinder: BOECKLEIN, Sebastian, 83052 Heufeld (DE); MESTL, Gerhard, 83052 Heufeld (DE); HOFMANN, Bernadette, 83052 Heufeld (DE); BINDSEIL, Gabriele, 83052 Heufeld (DE); ADLER, Anna, 83052 Heufeld (DE); RAMMEL, Nina, 83052 Heufeld (DE)
(74) Vertreter: Clariant Produkte (Deutschland) GmbH

(57) **Zusammenfassung**

Die Erfindung betrifft einen VPO-Katalysator in Form eines Formkörpers zur Oxidation von Kohlenwasserstoffen mit molekularem Sauerstoff, insbesondere zur Oxidation von Butan zu Maleinsäureanhydrid mit molekularem Sauerstoff, wobei der VPO-Katalysator zwischen 0,05 Gew.-% bis 7,0 Gew.-% Zn enthält, welches teilweise als ZnO vorliegt und bei in Transmission durchgeführter Infrarotspektroskopie eine erste Absorptionsbande mit einem Maximum zwischen 790 cm⁻¹ bis 810 cm⁻¹ und eventuell eine zweite Absorptionsbande mit einem Maximum zwischen 820 cm⁻¹ bis 840 cm⁻¹ aufweist, dadurch gekennzeichnet, dass nur die erste Absorptionsbande vorhanden ist oder die Stärke der ersten Absorptionsbande größer ist als die Stärke der zweiten Absorptionsbande. Die Erfindung betrifft des Weiteren ein Verfahren zur Herstellung eines erfindungsgemäßen VPO-Katalysators, umfassend die Schritte:
a) Herstellen einer Katalysatorvorstufe enthaltend Vanadylhydrogenphosphat,
b) Formen der Katalysatorvorstufe zu einem Formkörper.
c) Aktivieren der Formkörper, um eine VPO-Phase auszubilden,
dadurch gekennzeichnet, dass nach Schritt a) ZnO mit der Katalysatorvorstufe vermischt wird.

## Beschreibung

Die Erfindung betrifft einen VPO-Katalysator in Form eines Formkörpers zur Oxidation von Kohlenwasserstoffen mit molekularem Sauerstoff, insbesondere zur Oxidation von Butan zu Maleinsäureanhydrid mit molekularem Sauerstoff, wobei der VPO-Katalysator zwischen 0,05 Gew.-% bis 7,0 Gew.-% Zn enthält, welches teilweise als ZnO vorliegt und bei in Transmission durchgeführter Infrarotspektroskopie eine erste Absorptionsbande mit einem Maximum zwischen 790 cm⁻¹ bis 810 cm⁻¹ und eventuell eine zweite Absorptionsbande mit einem Maximum zwischen 820 cm⁻¹ bis 840 cm⁻¹ aufweist, dadurch gekennzeichnet, dass nur die erste Absorptionsbande vorhanden ist oder die Stärke der ersten Absorptionsbande größer ist als die Stärke der zweiten Absorptionsbande.

Die Erfindung betrifft auch ein Verfahren zur Herstellung eines erfindungsgemäßen VPO-Katalysators, umfassend die Schritte:
a) Herstellen einer Katalysatorvorstufe enthaltend Vanadylhydrogenphosphat,
b) Formen der Katalysatorvorstufe zu Formkörpern,
c) Aktivieren der Formkörper, um eine VPO-Phase auszubilden,
dadurch gekennzeichnet, dass nach Schritt a) ZnO mit der Katalysatorvorstufe vermischt wird.

Die Erfindung betrifft des Weiteren die Verwendung einer Zn-Verbindung in der Form eines Feststoffs zur Stabilisierung eines VPO-Katalysators in Partikelform.

Maleinsäureanhydrid ist ein chemisches Zwischenprodukt von großer wirtschaftlicher Bedeutung. Es wird beispielsweise bei der Herstellung von Alkyd- und Polyesterharzen allein oder auch in Kombination mit anderen Säuren eingesetzt. Darüber hinaus stellt es auch ein vielseitig einsetzbares Zwischenprodukt für die chemische Synthese dar, zum Beispiel für die Synthese von γ-Butyrolacton, Tetrahydrofuran und 1,4-Butandiol, welche wiederum ihrerseits als Lösungsmittel eingesetzt werden oder zu Polymeren, wie beispielsweise Polytetrahydrofuran oder Polyvinylpyrrolidon, weiterverarbeitet werden können.

Die Herstellung von Maleinsäureanhydrid erfolgt in der Regel durch partielle Oxidation von n-Butan in der Gasphase mit molekularem Sauerstoff oder mit einem molekularen Sauerstoff enthaltenden Gas in Gegenwart eines Vanadium-Phosphor-Oxid-Katalysators (VPO-Katalysators) der Vanadylpyrophosphat (VPP) enthält. Vanadylpyrophosphat weist in Reinform Vanadium mit einer Wertigkeit von + 4 auf und ist besonders geeignet für die Herstellung von Maleinsäureanhydrid aus unverzweigten gesättigten oder ungesättigten Kohlenwasserstoffen mit mindestens vier Kohlenstoffatomen. Zum Einsatz kommen sowohl Festbettreaktoren als auch Wirbelschichtreaktoren.

VPO-Katalysatoren weisen nur eine geringe intrinsische Aktivität bei der Reaktion von n-Butan zu Maleinsäureanhydrid auf. Daher ist für eine ausreichende Umwandlung eine große Menge an Katalysator erforderlich. Bei den VPO-Katalysatoren kommt noch hinzu, dass sie zu den teuersten Nicht-Edelmetallkatalysatoren überhaupt zählen, im Wesentlichen auf Grund der hohen Kosten ihrer Ausgangsmaterialien. Infolgedessen stellt sich die Aufgabe, die Katalyse-Performance (Aktivität und Selektivität) bzw. auch Lebensdauer und mechanischer Stabilität solcher Katalysatoren zu verbessern. Aus dem Stand der Technik ist bekannt, dass man die Performance der VPO-Katalysatoren durch die Beigabe von Fremdelementen zur Vanadium-Phosphor-Oxid-Phase (VPO-Phase) verbessern kann, wie zum Beispiel durch die Beigabe von Molybdän (Mo-Promotor oder Mo-Dotierung).

US 5,929,256 offenbart die Synthese eines aktiven mit Molybdän modifizierten Vanadium-Phosphor-Katalysators zur Herstellung von Maleinsäureanhydrid. Hierbei wird eine in wesentlichen Teilen 5-wertiges Vanadium enthaltende Verbindung mit einer 5-wertigen Phosphor enthaltenden Verbindung in einem alkoholischen Medium, das zur Reduktion des Vanadiums auf eine Oxidationsstufe unter 5 geeignet ist, umgesetzt. Hierbei wird Molybdän in das Reaktionsprodukt eingebaut, wobei eine feste, mit Molybdän modifizierte Vorläuferzusammensetzung gebildet wird. Der Alkohol wird entfernt, um eine getrocknete, feste mit Molybdän modifizierte Vorläuferzusammensetzung zu erhalten. Formkörper, die die getrocknete feste, mit Molybdän modifizierte Vorläuferverbindung enthalten, werden geformt. Die getrockneten und geformten mit Molybdän modifizierten Vorläuferzusammensetzungen werden aktiviert, um sie in den aktiven Katalysator umzuwandeln.

US 5,070,060 offenbart eine Verbesserung des Oxidationskatalysators, der für die partielle Oxidation von n-Butan verwendet wird und Vanadium- und Phosphor-, Zink- und Lithium-Mischoxide enthält, umfassend die Zugabe eines Molybdänverbindungs-Modifizierungsmittels in einer Menge von etwa 0,005 bis 0,025/1 Mo/V zum Katalysator während der Umsetzung der reduzierten Vanadiumverbindung mit konzentrierter Phosphorsäure. Die Zugabe von Mo erzeugt einen Katalysator, der sehr stabil ist, ein aktiveres System darstellt und länger haltbar ist als der unmodifizierte Katalysator.

US 3,980,585 offenbart einen Katalysator-Komplex, der sich für die Umwandlung von normalen C₄-Kohlenwasserstoffen zu Maleinsäureanhydrid in der Gasphase eignet, umfassend die Komponenten Vanadium, Phosphor und Kupfer sowie eines der Elemente ausgewählt aus der Gruppe Te, Zr, Ni, Ce, W, Pd, Ag, Mn, Cr, Zn, Mo, Re, Sm, La, Hf, Ta, Th, Co, U und Sn, vorzugsweise mit einem Alkalimetall oder einem Erdalkalimetall.

US 4,056,487 offenbart einen der für die Partialoxidation von Alkanen zu den entsprechenden Anhydriden, zum Beispiel von normalen C₄-Kohlenwasserstoffen die zu Maleinsäureanhydrid in der Gasphase umgewandelt werden, geeignet ist, umfassend die Komponenten Vanadium, Phosphor und Sauerstoff, Nb, Cu, Mo, Ni, Co und Cr. Bevorzugt sind die Zusammensetzungen, die zusätzlich eines oder mehrere Elemente aus Ce, Nd, Ba, Hf, U, Ru, Re, Li oder Mg enthalten.

US 4,515,904 offenbart ein Verfahren für die Herstellung eines Phosphor-Vanadium-Katalysators und eines Phosphor-Vanadium-Co-Metall-Katalysators für die Verwendung in der Herstellung von Maleinsäureanhydrid aus Butan, wobei das Verfahren, das Reagieren einer Vanadium-Verbindung in einem organischen-Ether-Lösungsmittel, das etwa 2 bis etwa 10 Kohlenstoffatome aufweist, mit einem Phosphor-Halogenid bei einer Temperatur von etwa 0 °C bis etwa 200 °C in der Anwesenheit von Wasser oder einem aliphatische Alkohol der etwa 1 bis etwa 8 Kohlenstoffatome aufweist; Entfernen des Lösungsmittels; und Aktivieren des Katalysators durch die Hinzugabe von Butan oder einem anderen Kohlenwasserstoff-Ausgangsprodukt und einer Phosphor-Verbindung bei einer Temperatur von ungefähr etwa 300 °C bis etwa 500 °C, umfasst.

US 5,158,923 offenbart eine Verbesserung des Oxidationskatalysators, der für die partielle Oxidation von n-Butan verwendet wird und Vanadium- und Phosphor-, Zink- und Lithium-Mischoxide enthält, umfassend die Zugabe eines Molybdänverbindungs-Modifizierungsmittels in einer Menge von etwa 0,005 bis 0,025 Mo/V zum Katalysator beim Aufschluss der reduzierten Vanadiumverbindung durch konzentrierte Phosphorsäure. Die Zugabe von Mo erzeugt einen Katalysator, der sehr stabil ist, ein aktiveres System darstellt und länger haltbar ist als der unmodifizierte Katalysator.

US 5,262,548 offenbart eine Verbesserung des Oxidationskatalysators, der für die partielle Oxidation von n-Butan verwendet wird und Vanadium- und Phosphor-, Zink- und Lithium-Mischoxide enthält, umfassend die Zugabe eines Molybdänverbindungs-Modifizierungsmittels in einer Menge von etwa 0,005 bis 0,025 Mo/V zum Katalysator beim Aufschluss der reduzierten Vanadiumverbindung durch konzentrierte Phosphorsäure. Die Zugabe von Mo erzeugt einen Katalysator, der ein sehr stabiles aktives System darstellt und der eine längere Lebensdauer aufweist als der unmodifizierte Katalysator.

WO 2013062919 A1 offenbart ein Verfahren zur Herstellung eines promotierten VPO-Katalysators, wobei der Katalysator die Mischoxide von Vanadium und Phosphor umfasst und wobei der Katalysator mit mindestens einem aus Niob, Kobalt, Eisen, Zink, Molybdän oder Titan promotiert wird, wobei das Verfahren die Schritte umfasst: (i) Herstellen eines VPO-Katalysators, umfassend Vanadylpyrophosphat als Hauptkomponente und enthaltend weniger als 5 Gew.-% Vanadylphosphat, (ii) Kontaktieren des VPO-Katalysators mit einer Lösung, umfassend eine Verbindung als Metallquelle mit mindestens einem Metall ausgewählt aus der Gruppe bestehend aus Niob, Kobalt, Eisen, Zink, Molybdän oder Titan, um einen metallimprägnierten VPO-Katalysator zu bilden, und (iii) Trocknen des metallimprägnierten VPO-Katalysators, um den promotierten VPO-Katalysator zu bilden. In einer Ausführungsform wird ein Niob-aktivierter VPO-Katalysator hergestellt.

US 5,280,003 offenbart eine Verbesserung eines Oxidationskatalysators, der für die partielle Oxidation von n-Butan verwendet wird und der Mischoxide aus Vanadium und Phosphor, Zink, Lithium und Molybdän enthält, umfassend dessen Herstellung unter Durchführung des Kristallisationsschrittes unter statischen Bedingungen, die gleichmäßigere Bedingungen für das Kristallwachstum ermöglichen. Die statischen Bedingungen werden aufrechterhalten, indem das Lösungsmittel während der Kristallisationsdauer unter Rückfluss erhitzt wird.

US 4,251,390 offenbart die Verbesserung eines für die partielle Oxidation von n-Butan verwendeten Oxidationskatalysators, der Vanadium und Phosphormischoxide enthält, dadurch gekennzeichnet, dass man dem Katalysator während der Umsetzung des reduzierten Vanadiumanteils mit konzentrierter Phosphorsäure eine Zinkverbindung in einer Menge von 0,15 bis 0,001 Zn/V zusetzt. Die Zugabe von Zink erzeugt einen Katalysator, der leichter aktiviert wird und der sehr stabil gegenüber einer Erwärmung des Reaktionssystems ist. Kleine Mengen an Lithium- und Siliziumverbindungen haben auch zusätzliche wünschenswerte katalytische Wirkungen, ohne den Vorteil der Zinkverbindung zu verringern.

DE 10 2014 004786 A1 betrifft einen Katalysator, der ein Vanadium-Phosphor-Oxid und ein Alkalimetall enthält, worin der Gewichtsanteil an Alkalimetall im Vanadium-Phosphor-Oxid im Bereich von 10 bis 400 ppm liegt, bezogen auf das Gesamtgewicht des Vanadium-Phosphor-Oxids, ein Verfahren zu dessen Herstellung sowie die Verwendung des Katalysators zur Gasphasenoxidation von Kohlenwasserstoffen, insbesondere zur Herstellung von Maleinsäureanhydrid.

Um VPO-Katalysatoren, welche eine VPP-Phase enthalten, herzustellen, wird üblicherweise eine Reduktion von Vanadiumpentoxid (V₂O₅) bei gleichzeitiger Anwesenheit von Phosphorsäure in einem organischen alkoholischen Lösungsmittel mit Benzylalkohol als Reduktionsmittel durchgeführt, wobei neben Benzaldehyd Vanadylhydrogenphosphat (VHP) entsteht. Die dabei ablaufende Redoxreaktion (die "Reduktion"), bei der Vanadium mit der Oxidationsstufe V (V(V)) zur VHP-Phase reagiert, in der Vanadylspezies (VO²⁺) mit Vanadium in der Oxidationsstufe IV (V(IV)) vorliegen, lautet:

(1) V₂O₅ + 2H₃PO₄ + Ph-CH₂-OH → 2VOHPO₄ * ½H₂O + Ph-CHO + 2H₂O

In einem darauffolgenden Aktivierungsschritt wird durch die Einwirkung von Wärme die VHP-Phase unter Abspaltung von Wasser zur Vanadylpyrophosphat-Phase umgewandelt.

(2) 2VOHPO₄ * ½H₂O → (VO)₂P₂O₇ + 1½H₂O

Bei der katalytischen Umsetzung von Butan zu Maleinsäureanhydrid mit VPO-Katalysatoren besteht eine Problematik darin, dass der Prozess im porendiffusionslimitierten Regime betrieben werden muss. Hier hat die Porosität dann direkten Einfluss auf die katalytische Ausbeute. Daher muss darauf geachtet werden, dass bei der Formgebung (z.B. durch Tablettieren) die Porenstruktur nicht negativ beeinflusst wird. Dies hat allerdings zur Folge, dass die mechanische Stabilität der Formkörper leidet. Diese muss jedoch so groß sein, dass die Tabletten den Füllprozess (Fall in ein ca. 6 m langes Reaktionsrohr) intakt überstehen. Andernfalls wäre durch die kleineren Bruchstücke der Staudruck im Prozess zu hoch, was zu hohen Kompressorkosten bzw. erniedrigten Durchsätzen führt. Eine technische Aufgabe besteht also darin, die mechanische Festigkeit der Formkörper zu erhöhen, ohne die Porenstruktur negativ zu beeinflussen. Gleichzeitig besteht das Bedürfnis nach VPO-Katalysatoren mit verbesserter Performance, d. h. verbesserter Aktivität, Selektivität und Stabilität.

Aufgabe der vorliegenden Erfindung war es daher, einen verbesserten VPO-Katalysator zur Gasphasenoxidation von Kohlenwasserstoffen, insbesondere zur Herstellung von Maleinsäureanhydrid bereitzustellen, der im Vergleich zu bisher üblichen Katalysatoren höhere katalytische Performance, insbesondere eine verbesserte Selektivität und gleichzeitig eine wesentlich verbesserte mechanische Festigkeit aufweist.

Die Aufgabe wird gelöst durch einen VPO-Katalysator in Form eines Formkörpers zur Oxidation von Kohlenwasserstoffen mit molekularem Sauerstoff, insbesondere zur Oxidation von Butan zu Maleinsäureanhydrid mit molekularem Sauerstoff, wobei der VPO-Katalysator zwischen 0,05 Gew.-% bis 7,0 Gew.-% Zn enthält, welches teilweise als ZnO vorliegt und bei in Transmission durchgeführter Infrarotspektroskopie eine erste Absorptionsbande mit einem Maximum zwischen 790 cm⁻¹ bis 810 cm⁻¹ und eventuell eine zweite Absorptionsbande mit einem Maximum zwischen 820 cm⁻¹ bis 840 cm⁻¹ aufweist, dadurch gekennzeichnet, dass nur die erste Absorptionsbande vorhanden ist oder die Stärke der ersten Absorptionsbande größer ist als die Stärke der zweiten Absorptionsbande.

Die Stärke der Absorptionsbande wird hierbei bestimmt, indem jeweils eine erste Gerade so gesetzt wird, dass diese das Spektrum unmittelbar links und rechts unterhalb der betreffenden Bande tangential berührt, anschließend eine zweite Gerade ausgehend vom Maximum der Bande in Richtung gleichbleibender Wellenlänge und abnehmender Absorption geführt wird und der Schnittpunkt der beiden Geraden bestimmt wird, wobei die Stärke der Absorptionsbande definiert ist als die Länge zwischen Maximum und Schnittpunkt. Vorzugsweise liegt hierbei das Verhältnis der Stärke der ersten Absorptionsbande zur Stärke der zweiten Absorptionsbande über 1,1 vorzugsweise über 2.

Die erfindungsgemäßen Formkörper weisen eine Seitendruckfestigkeit von mehr als 25 N, vorzugsweise zwischen 25 N bis 200 N auf, wobei die Seitendruckfestigkeit mit einem Zwick Z0.5 Gerät unter Verwendung der Norm ASTM D4179 mit einer konstanten Kraftrate von 20,0 N/s gemessen wurden, wobei 100 Tabletten jeweils einzeln mit der Zylinderachse parallel zur Messbackenoberfläche platziert und vermessen wurden, um eine durchschnittliche Bruchkraft zu bestimmen, die die Seitendruckfestigkeit darstellt.

Der erfindungsgemäße VPO-Katalysator weist, wenn er mit Pulver-Röntgendiffraktometrie unter Verwendung der Cu-Ka-Strahlung untersucht wird, Reflexe bei 31,7 ° bis 31,9 °, 34,3 ° bis 34,5 ° und 36,2 ° bis 36,4 ° auf.

Der erfindungsgemäße VPO-Katalysator umfasst die VPO-Phase, bzw. besteht oder besteht im Wesentlichen aus ihr. Zum Beispiel umfasst der erfindungsgemäße VPO-Katalysator die VPO-Phase mit einem Gehalt von mehr als 70 Gew.-%, bevorzugt mehr als 80 Gew.-%, stärker bevorzugt mehr als 90 Gew.-%, bezogen auf das Gesamtgewicht des VPO-Katalysators. Im Übrigen kann der VPO-Katalysator, VPP, Dotierungen und nicht umgesetzte Oxide der Ausgangsstoffe umfassen, wie zum Beispiel Vanadiumpentoxid oder Phosphoroxid.

Der erfindungsgemäße VPO-Katalysator kann optional zum Beispiel zwischen 0,1 Gew.-% und 1 Gew.-%, bevorzugter Weise zwischen 0,4 Gew.-% und 0,7 Gew.-% Mo enthalten, bezogen auf das Gesamtgewicht des VPO-Katalysators.

Der erfindungsgemäße VPO-Katalysator kann aber auch Alkalimetalle wie Na, K, enthalten, vorzugsweise 80 bis 300 ppm Alkalimetall. Darüber hinaus kann der erfindungsgemäße VPO-Katalysator auch Kohlenstoff, z.B. in Form von Graphit, enthalten, z.B. in einer Menge von 3 Gew.-% bis 5 Gew.-%, bezogen auf das Gesamtgewicht des Katalysators. Der anwesende Graphit dient z.B. als Tablettierhilfsmittel.

Der erfindungsgemäße VPO-Katalysator weist ZnO auf, kann aber noch weitere Zn-Verbindungen enthalten. Der Gehalt an Zn der sich durch die Anwesenheit von ZnO und möglicherweise vorhandenen weiteren Zn-Verbindungen ergibt, muss im VPO-Katalysator zwischen 0,05 Gew.-% und 7,0 Gew.-%, bevorzugterweise zwischen 0,1 Gew.-% und 6,0 Gew.-%, stärker bevorzugt zwischen 0,2 Gew.-% und 4,0 Gew.-%, am stärksten bevorzugt zwischen 0,7 Gew.-% und 3,0 Gew.-% Zn sein, jeweils bezogen auf das Gesamtgewicht des Katalysators.

Der VPO-Katalysator weist bevorzugter Weise folgende elementare Zusammensetzung auf:
- 0,05 Gew.-% bis 7 Gew.-% Zn,
- 0 Gew.-% bis 0,7 Gew.-% Mo,
- 26 Gew.-% bis 31 Gew.-% V,
- 17 Gew.-% bis 21 Gew.-% P,
- 3 Gew.-% bis 5 Gew.-% C,
- im übrigen Sauerstoff, jeweils bezogen auf das Gesamtgewicht des VPO-Katalysators.

Der erfindungsgemäße VPO-Katalysator weist ZnO auf und sofern der Gehalt an diesen beiden Phasen ausreichend hoch ist, weist der Katalysator in einem XRD-Pulverdiffraktogramm, das unter Verwendung der Cu-Ka-Strahlung aufgenommen worden ist, Reflexe auf, die typisch für die ZnO-Phase sind. Insbesondere werden scharfe Reflexe bei 31,7° bis 31.9°, 34,3° bis 34,5°, 36,2° bis 36,4° detektiert.

Die Anwesenheit von ZnO bewirkt erfindungsgemäß eine Stabilisierung der Katalysatorpartikel, sodass sie im Vergleich zur Festigkeit ohne das Vorhandensein von ZnO eine höhere mechanische Festigkeit aufweisen. Die Anwesenheit von ZnO bewirkt aber auch eine Verbesserung der katalytischen Performance (d. h. der Aktivität, Selektivität und Stabilität) der Katalysatorpartikel, insbesondere eine Erhöhung der Selektivität.

Der erfindungsgemäße VPO-Katalysator liegt als Formkörper vor, die Form des Formkörpers kann unterschiedlich gestaltet werden, je nach gewünschter Kontaktzeit, Durchflussgeschwindigkeit und Staudruck bei der katalytischen Umsetzung. Unter dem VPO-Katalysator in Form eines Formkörpers werden Formkörper verstanden, die durch einen Formungsschritt wie Tablettierung hergestellt werden. Eine Vielzahl der erfindungsgemäßen Formkörper bilden im Rohrbündelreaktor eine Lage oder ein Katalysatorbett, durch das die Edukte Butan, insbesondere n-Butan und Luft geleitet werden.

Zum Beispiel kann der erfindungsgemäße Formkörper in einer üblichen Zylinder-Form vorliegen. Der Zylinder weist dann eine Höhe (Länge längs der Zylinderachse) von 3 mm bis 8 mm und eine im Wesentlichen runde Grundfläche mit einem Durchmesser von 3 mm bis 8 mm auf. Bevorzugterweise weist der Zylinder eine mittlere axiale Öffnung auf, diese kann zum Beispiel einen Durchmesser von 1 mm bis 3 mm aufweisen.

Zum Beispiel kann der erfindungsgemäße Formkörper eine Höhe von 4,7 mm, einem Außendurchmesser von 4,7 mm und einer mittleren axialen Öffnung mit einem Durchmesser von 1,3 mm aufweisen. Dieser Formkörper weist dann eine geometrische Oberfläche von 1,2 cm², ein Volumen von 0,075 cm³ und eine Masse von 0,12 g auf. Bei dem Einfüllen einer Vielzahl dieser Formkörper in einen 21 mm Reaktor ergibt sich eine Fülldichte von 0,85 g/cm³ bis 0,89 g/cm³.

Zum Beispiel können die erfindungsgemäßen Formkörper eine Höhe von 5,6 mm, einem Außendurchmesser von 5,5 mm und einer mittleren axialen Öffnung mit einem Durchmesser von 2,3 mm aufweisen. Diese Formkörper weisen dann eine geometrische Oberfläche von 1,77 cm², ein Volumen von 0,111 cm³ und eine Masse von 0,18 g auf. Bei dem Einfüllen einer Vielzahl dieser Formkörper in einen 21 mm Reaktor ergibt sich eine Fülldichte von 0,72 g/cm³ bis 0,76 g/cm³.

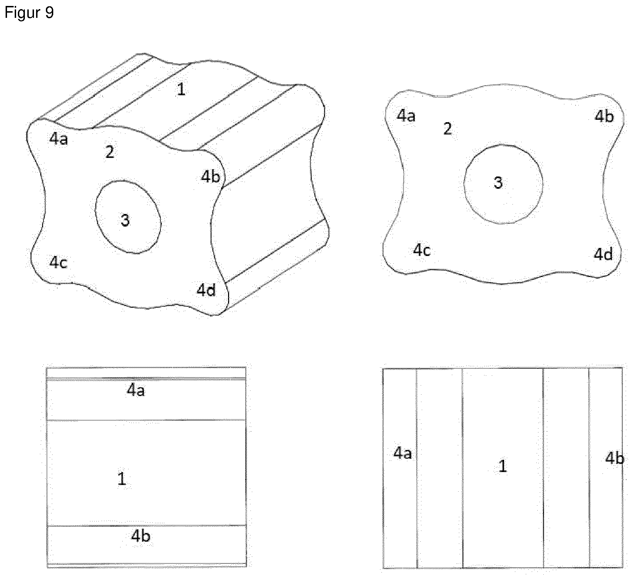

Bevorzugte Formkörper zur Verwendung in dem erfindungsgemäßen Reaktorkonzept sind die, die in EP 2643086 A1 beschrieben sind. Die bevorzugte Doppelalphaform ist insbesondere dadurch gekennzeichnet, dass jeder einzelne Formkörper jeweils als Zylinder mit einer Außengrundfläche [1], einer Zylinderfläche [2], einer Zylinderachse und mindestens einer durchgehenden, parallel zur Zylinderachse verlaufenden Öffnung [3] ausgebildet ist und die Außengrundfläche [1] des Zylinders mindestens vier Loben [4a, 4b, 4c, 4d] aufweist, wobei ein die Formkörper umschließender geometrische Grundkörper ein Prisma ist, das eine Prismengrundfläche mit einer Länge und einer Breite aufweist, wobei die Länge größer ist als die Breite und wobei die Loben [4a, 4b, 4c, 4d] von Prismenecken der Prismengrundfläche umschlossen sind (Figur 9).

Bevorzugt ist ein Formkörper in einer Doppelalphaform mit einer Höhe (Länge längs der Zylinderachse) von 3 mm bis 8 mm, einer Länge von 5 mm bis 9 mm und einer Breite von 4 mm bis 8 mm und einem Lochinnendurchmesser von 1 mm bis 4 mm. Bevorzugt sind zum Beispiel Formkörper in einer Doppelalphaform mit einer Höhe von 5,6 mm, einer Länge von 6,7 mm, einer Breite von 5,8 mm und einem Lochinnendurchmesser von 2,1 mm. Diese Katalysatorformkörper weisen eine geometrische Oberfläche von 2,37 cm², ein Volumen von 0,154 cm³ und eine Masse von 0,24 g auf. Bei dem Einfüllen in einen 21 mm Reaktor ergibt sich eine Fülldichte von 0,60 g/cm³ bis 0,62 g/cm³.

Der erfindungsgemäße VPO-Katalysator bzw. der Formkörper hat eine Seitendruckfestigkeit von mehr als 25 N, vorzugsweise zwischen 25 N bis 200 N. Insbesondere bevorzugt ist eine Seitendruckfestigkeit des VPO-Katalysators bzw. des Formkörpers von mehr als 30 N bis 150 N, noch stärker bevorzugt von mehr als 35 N bis 100 N.

Der erfindungsgemäße VPO-Katalysator bzw. der Formkörper in Zylinderform hat eine Seitendruckfestigkeit von mehr als 25 N, vorzugsweise zwischen 25 N bis 50 N. Insbesondere bevorzugt ist eine Seitendruckfestigkeit des VPO-Katalysators bzw. des Formkörpers von mehr als 30 N bis 45 N, noch stärker bevorzugt von mehr als 35 N bis 40 N.

Der erfindungsgemäße VPO-Katalysator bzw. der Formkörper in Doppelalphaform hat eine Seitendruckfestigkeit von mehr als 50 N, vorzugsweise zwischen 50 N bis 200 N. Insbesondere bevorzugt ist eine Seitendruckfestigkeit des VPO-Katalysators bzw. des Formkörpers von mehr als 100 N bis 170 N, noch stärker bevorzugt von mehr als 120 N bis 150 N.

Die Erfindung betrifft des Weiteren ein Verfahren zur Herstellung eines erfindungsgemäßen VPO-Katalysators, umfassend die Schritte:
a) Herstellen einer Katalysatorvorstufe enthaltend Vanadylhydrogenphosphat,
b) Formen der Katalysatorvorstufe zu Formkörpern,
c) Aktivieren der Formkörper, um eine VPO-Phase auszubilden,
   dadurch gekennzeichnet, dass nach Schritt a) ZnO mit der Katalysatorvorstufe vermischt wird.

In einem Verfahrensschritt a) wird in allgemein bekannter Weise eine Katalysatorvorstufe hergestellt, die Vanadylhydrogenphosphat enthält. Typischerweise wird hierbei in einer Reaktionsmischung eine V(V)-Verbindung in Lösung mithilfe eines Reduktionsmittel unter der Anwesenheit einer P(V)-Verbindung, optional eine Mo-Verbindung, in einem Reduktionsschritt reduziert. Zum Beispiel kann die Reaktionsmischung aus 45 Gew.-% bis 90 Gew.-% Lösungsmittel, 5 Gew.-% bis 15 Gew.-% Reduktionsmittel, 5 Gew.-% bis 15 Gew.-% V(V)-Verbindung, bis zu 1 Gew.-% Mo-Verbindung und zwischen 5 bis 25 Gew.-% P(V)-Verbindung bestehen.

Konkreter können zum Beispiel 60 Gew.-% bis 70 Gew.-% Isobutanol, 5 Gew.-% bis 15 Gew.% Benzylalkohol, 5 Gew.-% bis 15 Gew.-% Vanadiumpentoxid, 0,05 Gew.-% bis 0,2 Gew.-% (NH₄)₂MO₂O₇ und 10 Gew.-% bis 20 Gew.-% Phosphorsäure, jeweils bezogen auf das Gesamtgewicht der Reaktionsmischung, als Reaktionsmischung vorgelegt werden.

Die V(V)-Verbindung, die als Ausgangsmaterial in der Reaktionsmischung zur Herstellung des Vanadylhydrogenphosphat verwendet wird, ist eine Verbindung, die Vanadium in der Oxidationsstufe V enthält und ist bevorzugt V₂O₅. Die P(V)-Verbindung, die als Ausgangsmaterial in der Reaktionsmischung zur Herstellung des Vanadylhydrogenphosphat verwendet wird, ist eine Verbindung, die Phosphor in der Oxidationsstufe V enthält und ist bevorzugt Phosphorsäure, oder ein Phosphatsalz, wie Na₃PO₄. Wird Phosphorsäure (H₃PO₄) eingesetzt, ist diese vorzugsweise wasserfrei (100-prozentige Phosphorsäure) oder Phosphorsäure, die nur geringe Mengen an Wasser enthält, d.h. Phosphorsäure mit einer Konzentration von 98 bis 100%, bevorzugt 99 bis 100% (die Angabe bezieht sich auf den üblicherweise angegebenen prozentualen Gewichtsanteil der reinen Phosphorsäure in Bezug zu dem Gewicht der Wasser-Phosphorsäuremischung). Alternativ kann bei der Herstellung der Reaktionsmischung zur Herstellung des Vanadylhydrogenphosphats Phosphorsäure mit mehr als 100 % eingesetzt werden, die mit dem in der Reaktionsmischung zu Beginn eventuell vorhandenen Wasser sofort zu Phosphorsäure mit einer Konzentration von 98 bis 100 %, bevorzugt 99 bis 100 %, vorzugsweise 100 % reagiert, so dass in der Reaktionsmischung keine Phosphorsäure mit mehr als 100 % Konzentration vorliegt und gleichzeitig nicht mehr als 0,2 Gew.-% Wasser, bezogen auf das Gewicht der Reaktionsmischung, in der Reaktionsmischung verbleibt.

Die Mo-Verbindung, die optional als Ausgangsmaterial in der Reaktionsmischung zur Herstellung des Vanadylhydrogenphosphats verwendet werden kann, ist eine beliebige Verbindung die Molybdän enthält, zum Beispiel Molybdäntrioxid, Ammoniumheptamolybdat ((NH₄)₆Mo₇O₂₄)*4H₂O), Ammoniumparamolybdat ((NH₄)₆Mo₇O₂*4H₂O), meta Molybdat, Molybdänsäure (H₂MoO₄) sowie deren Salze wie, (NH₄)₂MoO₄, Na₂MoO₄, K₂MoO₄ oder (NH₄)₂Mo₂O₇.

Das Reduktionsmittel, das in der Reaktionsmischung zur Herstellung des Vanadylhydrogenphosphats anwesend ist, kann jedes beliebige Reduktionsmittel sein, das in der Lage ist, die V(V)-Verbindung zu reduzieren, so dass zumindest teilweise Vanadylhydrogenphosphat entsteht. Vorzugsweise ist das Reduktionsmittel ein organisches Reduktionsmittel, wie Ethanol, Isobutanol oder ein aromatischer Alkohol, darunter insbesondere Benzylalkohol.

Das Lösungsmittel, das in der Reaktionsmischung zur Herstellung des Vanadylhydrogenphosphats anwesend ist, ist vorzugsweise ein Alkohol, besonders bevorzugterweise ein hochsiedender aliphatischer Alkohol, insbesondere Isobutanol, alternativ Ethanol oder iso-Propanol.

Die Ausgangsmaterialien werden in einem geeigneten Reaktionsbehältnis bereitgestellt, um den Reduktionsschritt durchzuführen und um das Vanadylhydrogenphosphat der Katalysatorvorstufe zu erhalten. Die Reaktionsmischung wird hierbei oberhalb Raumtemperatur erwärmt, zum Beispiel bis zu einer Temperatur von 100 °C. Nachdem die Reduktion vorzugsweise unter Rühren in einem Refluxschritt durchgeführt wird, weist das Reaktionsbehältnis vorzugsweise einen Rückflusskühler und eine Vorrichtung zum Rühren der Reaktionsmischung auf. Optional ist das Reaktionsbehältnis mit einer Vorrichtung ausgestattet, die es erlaubt, das während der Reduktion entstehende Wasser aus der Reaktionsmischung zu entfernen, das heißt einen Wasserabscheider, wie zum Beispiel eine Dean Stark-Falle.

Die Reduktion zu Vanadylhydrogenphosphat erfolgt vorzugsweise unter Reflux bei Normaldruck, hierbei ist die Temperatur in Abhängigkeit des Siedepunkts des verwendeten Lösungsmittels erhöht, bevorzugterweise wird ausschließlich ein einziger Refluxschritt in dem erfindungsgemäßen Verfahren durchgeführt. Die Katalysatorvorstufe enthält vorzugsweise als Hauptphase Vanadylhydrogenphosphat oder kann sogar im Wesentlichen aus einer Vanadylhydrogenphosphat-Phase bestehen. Das in der Katalysatorvorstufe möglicherweise ebenfalls anwesende Molybdän kann als Dotierung der Vanadylhydrogenphosphat-Phase vorliegen, wobei unter Molybdändotierung zu verstehen ist, dass das Molybdän entweder in die Vanadylhydrogenphosphat-Phase eingebaut wird oder auf dessen Oberfläche vorliegt. Neben der Vanadylhydrogenphosphat-Phase können bei der Reduktion jedoch noch weitere Vanadium-Phosphor-Mischoxide entstehen, in denen Vanadium eine Oxidationsstufe von IV oder sogar III aufweist. Die Reduktion muss nicht vollständig verlaufen, so dass auch Anteile der V(V)-Verbindung und der P(V)-Verbindung in der Katalysatorvorstufe verbleiben. Typischerweise weist die Katalysatorvorstufe aber eine mittlere Oxidationsstufe des Vanadiums von 3,8 bis 4,2 auf.

Das während der Reduktion entstehende Wasser kann während der Reduktion aus der Reaktionsmischung entfernt werden. Die Entfernung des Wassers während der Reduktion erfolgt im Stand der Technik entweder physikalisch, z.B. mit Hilfe eines Wasserabscheiders, oder chemisch durch die Verwendung von Verbindungen, die das Wasser binden, also z.B. Trocknungsmittel oder Anhydride wie Phosphorsäure mit mehr als 100 % Konzentration. Die Reaktionsmischung kann z.B. Anhydride aufweisen, die mit Wasser reagieren und dieses binden, insbesondere kann die Reaktionsmischung Phosphorsäure mit mehr als 100 % Konzentration aufweisen.

Die nach dem Reduktionschritt erhaltene Suspension kann zum Beispiel in einer InertgasAtmosphäre wie Stickstoff oder ein Edelgas filtriert werden. In diesem Zusammenhang bedeutet Inertgas jedes Gas, das unter den gegebenen Bedingungen bei der Filtration nicht mit der Katalysatorvorstufe reagiert, gleichzeitig aber Sauerstoff aus der Luft verdrängt, so dass die Explosionsgefahr minimiert wird. Das Filtrieren erfolgt durch die dem Fachmann bekannten Mittel, typischerweise durch eine Filterpresse, einen Dekanter oder durch eine Nutsche. Durch das Filtrieren wird eine unkalzinierte Katalysatorvorstufe erhalten, welches noch mit Lösungsmittel durchfeuchtet ist.

Die durch die Filtration erhaltene Katalysatorvorstufe (der feste Rückstand der Filtration) kann im Folgenden getrocknet werden, dies erfolgt typischerweise bei einer Temperatur oberhalb der Raumtemperatur, z.B. bei einer Temperatur bis zu 150 °C bei reduziertem Druck, bzw. Vakuum oder unter Inertgas, um eine getrocknete Katalysatorvorstufe zu erhalten. In diesem Zusammenhang bedeutet Inertgas, jedes Gas, welches unter den Bedingungen der Trocknung nicht mit der Katalysatorvorstufe reagiert, gleichzeitig aber Sauerstoff aus der Luft verdrängt, so dass die Explosionsgefahr minimiert wird, zum Beispiel Stickstoff oder ein Edelgas. Bevorzugterweise verläuft das Trocknen unter reduziertem Druck oder Vakuum zwischen 50 °C und 150 °C, vorzugsweise zwischen 90 °C und 140 °C.

Alternativ oder optional folgend auf das Trocknen, kann eine Kalzinierung erfolgen, um die Katalysatorvorstufe zu erhalten. Die Kalzinierung erfolgt bei erhöhter Temperatur zwischen 150 °C und 350 °C, vorzugsweise zwischen 230 °C und 290 °C unter Inertgas. In diesem Zusammenhang bedeutet Inertgas, jedes Gas, welches unter den Bedingungen der Kalzinierung nicht mit der Katalysatorvorstufe reagiert aber Sauerstoff aus der Luft verdrängt, so dass die Explosionsgefahr minimiert wird, zum Beispiel Stickstoff oder ein Edelgas.

Optional kann Graphit zu der getrockneten Katalysatorvorstufe beigemischt werden, um die Formung in Verfahrensschritt b) zu erleichtern. Es kann auch ein Kompaktieren und/oder Granulieren des getrockneten Katalysatorvorstufe erfolgen, um eine kompaktierte oder granulierte getrocknetes Katalysatorvorstufe zu erhalten. Hierbei kann die Katalysatorvorstufe mit einem Walzenkompaktor mit einem Anpressdruck von 190 bar, einer Spaltbreite 0,60 mm und einer Walzengeschwindigkeit von 7 U/min, zu Platten kompaktiert werden und durch ein 1 mm Sieb granuliert werden.

Im Verfahrensschritt b) wird die erhaltene Katalysatorvorstufe zu den Katalysatorformkörpern geformt, dies kann zum Beispiel durch eine Tablettierung erfolgen. Hierbei wird z.B. das Granulat mit einer Rundläufertablettenpresse zu der gewünschten Tablettenform, mit entsprechender Größe gepresst.

Im folgenden Verfahrensschritt c) erfolgt das Aktivieren der erhaltenen Katalysatorformkörper, bei einer Temperatur oberhalb von 200 °C. Die Aktivierung erfolgt typischerweise in einer Gasmischung bestehend aus Luft, Inertgas und Wasserdampf, wobei als Inertgas jedes Gas dienen kann, das unter den gegebenen Bedingungen bei der Aktivierung nicht mit dem Katalysatorformkörper reagiert, besonders bevorzugt ist das Inertgas Stickstoff oder ein Edelgas. Alternativ kann die Aktivierung auch in Prozessgas erfolgen, d.h. in einem Luft und Butan enthaltenden Gasgemisch. Die Aktivierung erfolgt bei einer Temperatur im Bereich von 300 °C bis 500 °C, vorzugsweise im Bereich von 350 °C bis 450 °C. Mit der Aktivierung wird der fertige VPO-Katalysator als Produkt des Verfahrens erhalten. Der tablettierte VPO-Katalysator hat typischerweise eine Seitendruckfestigkeit von oberhalb 25 N, typischerweise zwischen 25 N bis 200 N.

Das erfindungsgemäße Herstellungsverfahren des VPO-Katalysators in Form von Formkörpern ist dadurch gekennzeichnet, dass nach Verfahrensschritt a) ZnO zu der Katalysatorvorstufe zugegeben wird, bzw. mit dieser vermischt wird. Das ZnO, d.h. Zink(II)-oxid, wird hierbei als Pulververmittler-Feststoff verwendet. Die Zugabe des ZnO zu der Katalysatorvorstufe kann auch mit Hilfe einer Dosiervorrichtung und eines Mischers erfolgen. Generell wird ausreichend ZnO zugegeben, um den erfindungsgemäßen Katalysator darzustellen. Zum Beispiel können bezogen auf das Gewicht der getrockneten Katalysatorvorstufe (ohne ZnO) 0,5 Gew.-% bis 7,0 Gew.-%, bevorzugter Weise 1,0 Gew.-% bis 5,0 Gew.-%, stärker bevorzugt 1,5 Gew.-% bis 3,5 Gew.-% ZnO in fester Form zugegeben werden.

Die Zugabe des ZnO erfolgt zu der Katalysatorvorstufe, d. h. nach dessen Herstellung in Verfahrensschritt a) zum Beispiel durch einen Reduktionsschritt, jedoch vor der Formung der Katalysatorformkörper in Verfahrensschritt c). Vorzugsweise, erfolgt die Zugabe des ZnO zu der getrockneten Katalysatorvorstufe, besonders bevorzugt zu der getrockneten und kalzinierten Katalysatorvorstufe.

In einer weiteren bevorzugten Ausführungsform wird neben dem ZnO eine weitere feste Metall-Verbindung, vorzugsweise eine Metall-Verbindung in Pulverform zugegeben. Bevorzugt ist, dass eine feste Mg-Verbindung mit in dem Bindemittel enthalten ist, zum Beispiel MgO.

Die Erfindung betrifft auch die Verwendung von ZnO zur Stabilisierung eines VPO-Katalysators in Form eines Formkörpers sowie die Verwendung von ZnO als Bindemittel für einen VPO-Katalysator in Form eines Formkörpers.

Die Erfindung betrifft des Weiteren ein Verfahren zur Herstellung von Maleinsäureanhydrid durch katalytische Oxidation von n-Butan, wobei ein Eduktgas umfassend Sauerstoff und n-Butan durch eine Reaktorröhre geleitet wird, in der sich eine Schüttung von erfindungsgemäßen VPO-Katalysatoren befindet.

Die Schüttung der VPO-Katalysatoren in der Reaktorröhre besteht aus den erfindungsgemäßen VPO-Katalysatoren bzw. Formkörpern, die in die Reaktorröhre eingegeben werden und durch Aufliegen eine Schüttung ergeben. Die Reaktorröhre ist vorzugsweise Teil einer Vielzahl von Reaktorröhren eines Rohrbündelreaktors, wie er dem Fachmann zur industriellen Herstellung von Maleinsäureanhydrid bekannt ist. Während der Reaktion liegt die Schüttung der VPO-Katalysatoren bei einer Temperatur zwischen 300 °C und 420 °C vor. Das Eduktgas kann zum Beispiel zwischen 0,2 bis 10 Vol.-% n-Butan und zwischen 5 und 50 Vol.-% Sauerstoff enthalten und mit einer Raumzeitgeschwindigkeit von 1100 h⁻¹ bis 2500 h⁻¹, vorzugsweise 1300 h⁻¹ bis 2000 h⁻¹ durch die Reaktorröhre geleitet werden.
Figur 1: IR-Spektren der Proben gemäß den Beispielen 1 bis 7 und Beispiel 12.
Figur 2: IR-Spektren der Proben gemäß den Beispielen 1 und 15 und 16.
Figur 3: IR-Spektren der Proben gemäß dem Beispiel 5 und den Vergleichsbeispielen 17 und 18.

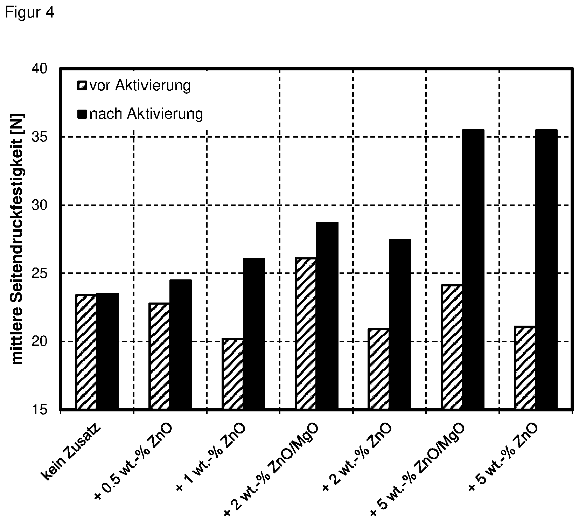

Figur 4: Einfluss der Verwendung von ZnO bzw. ZnO/MgO-Feststoffen bei der Synthese auf die Stabilität der VPO-Katalysatoren nach den Beispielen 1 bis 7.
Figur 5: Veränderung der Seitendruckfestigkeit und Selektivität der VPO-Katalysatoren bei unterschiedlichen Mengen an Zn-Zusatz.
Figur 6: XRD-Diffraktogramme der VPO-Katalysatoren nach den Beispielen 1 bis 5.
Figur 7: XRD-Diffraktogramme der VPO-Katalysatoren nach den Beispielen 5 und 8 bis 10.
Figur 8: XRD-Diffraktogramme der VPO-Katalysatoren gemäß dem Beispiel 11 und 12 im Vergleich zu dem die gemäß der Erfindung hergestellten VPO-Katalysator nach Beispiel 5.
Figur 9: Darstellungen des bevorzugten Katalysatorpartikels, die "Doppelalpha-Form" aus vier verschiedenen Perspektiven.

Die Ziffern in Klammern in den Figuren verweisen jeweils auf die nach dem entsprechenden Beispiel hergestellte Probe.

### Beispiele

### Beispiel 1 (Vergleich)

### Verwendete Apparatur

Auf einen Laborboy wird ein Heizpilz gestellt und in diesem befindet sich ein 2L-Vierhalskolben. In der mittleren Öffnung des Vierhalskolbens befindet sich ein Halbmondrührer mit passendem Rührverschluss, der mittels einer Rührerkupplung an das Rührwerk angeschlossen ist. In der rechten Öffnung befindet sich ein Thermometer, in der Linken ein Steigrohr zum Rückflusskühler. Die Öffnung vorne in der Mitte wird zum Befüllen mit den Chemikalien verwendet, danach wird die Stickstoffspülung dort angeschlossen. Die gesamte Apparatur kann auch mit Stickstoff geflutet werden. Hierfür wird der Stickstoff als erstes durch eine Gaswaschflasche und dann in die Apparatur geleitet und oben aus dem Kühler wiederum durch eine Gaswaschflasche ausgeleitet.

### Herstellen der Katalysatorvorstufe

Als erstes werden 1069,5 g Isobutanol und 156,0 g Benzylalkohol zugegeben. Unter Rühren erfolgt die Zugabe von 150 g V₂O₅. Nach der V₂O₅ Zugabe erfolgt die Zugabe von 2,52 g Ammoniumdimolybdat. Anschließend werden 232,50 g Phosphorsäure (100 %, bzw. wasserfrei) zur Suspension zugegeben und unter N₂ im Rückfluss für 10 h geheizt.

### Filtrieren

Nach Abkühlen der Suspension, die das Katalysatorvorprodukt als Feststoff enthält, wird diese aus dem Vierhalskolben in eine Filternutsche übertragen und die Flüssigkeit abgesaugt. Der feuchte Filterkuchen wird in einer Presse über Nacht bei 14 bis 18 bar trocken gepresst.

### Trocknung / Kalzinierung

Der ausgepresste Filterkuchen wird in den Verdampferkolben eines Rotationsverdampfers gefüllt. Unter Wasserstrahlvakuum wird der Filterkuchen bei 110 °C über Nacht getrocknet. Das so getrocknete Pulver wird in einem geeigneten Kalziniertopf in einen Ofen gestellt und in einer N₂-Atmosphäre bei Temperaturen von 200 bis 300 °C für 9 h kalziniert.

### Kompaktierung/Tablettierung

Vor der Kompaktierung/Tablettierung werden dem kalzinierten pulverförmigen Katalysatorvorprodukt 5 Gew.-% Graphit zugegeben und mit Hilfe eines Rhönradmischers homogen durchmischt. Dieses Pulver wird mit einem Walzenkompaktor mit einem Anpressdruck von 190 bar, einer Spaltbreite 0,60 mm und einer Walzengeschwindigkeit von 7 U/min, zu Platten kompaktiert und durch ein 1 mm Sieb granuliert.

Das Granulat wird mit einer Rundläufertablettenpresse zu der gewünschten Tablettenform, mit entsprechender Höhe, z.B. 5,6 x 5,6 x 2,3 mm, und Seitendruckfestigkeit, gepresst.

### Aktivierung zum Pyrophosphat

Die Aktivierung, bei der Vanadylpyrophosphat entsteht, wird in einer in einem programmierbaren Ofen eingebauten Retorte unter kontrollierten Bedingungen durchgeführt. Die kalzinierten Tabletten werden gleichmäßig in die Retorte eingefüllt und diese wird dicht verschlossen. Danach wird der Katalysator in einer feuchten Luft-Stickstoffmischung (50% absolute Luftfeuchtigkeit) zuerst bei über 300 °C für 5 h anschließend bei über 400 °C für 9 h aktiviert.

### Beispiele 2 bis 7 (erfindungsgemäß)

Die erfindungsgemäßen VPO-Katalysatoren wurden analog zu Beispiel 1 hergestellt, mit der Ausnahme, dass das kalzinierte Pulver der Katalysatorvorstufe pro 100 g mit 4,5 g (Beispiel 2), 1,8 g (Beispiel 3), 5,0 g (Beispiel 4), 2,0 g (Beispiel 5), 1,0 g (Beispiel 6), 0,5 g (Beispiel 7) ZnO-Pulver (kommerzielles ZnO Partikelgröße < 100 µm, Verunreinigung unter 0,05 Gew.-%) und 0,5 g (Beispiel 2) und 0,2 g (Beispiel 3) MgO-Pulver und gleichzeitig mit dem Graphit vermengt wurde. Damit ergaben sich kalzinierte Vorläufer-Pulver mit 5,0 Gew.-% ZnO/MgO (Beispiel 2), 2,0 Gew.-% ZnO/MgO (Beispiel 3), 5,0 Gew.-% ZnO (Beispiel 4), 2,0 Gew.-% ZnO (Beispiel 5), 1,0 Gew.-% ZnO (Beispiel 6), 0,5 Gew.-% ZnO (Beispiel 7).

Die VPO-Katalysatoren nach den Beispielen 1 bis 7 wurden jeweils vor und nach der Aktivierung auf ihre Bruchfestigkeit getestet. Außerdem wurden XRD-Diffraktogramme der VPO-Katalysatoren erstellt und diese auf ihre katalytische Selektivität getestet.

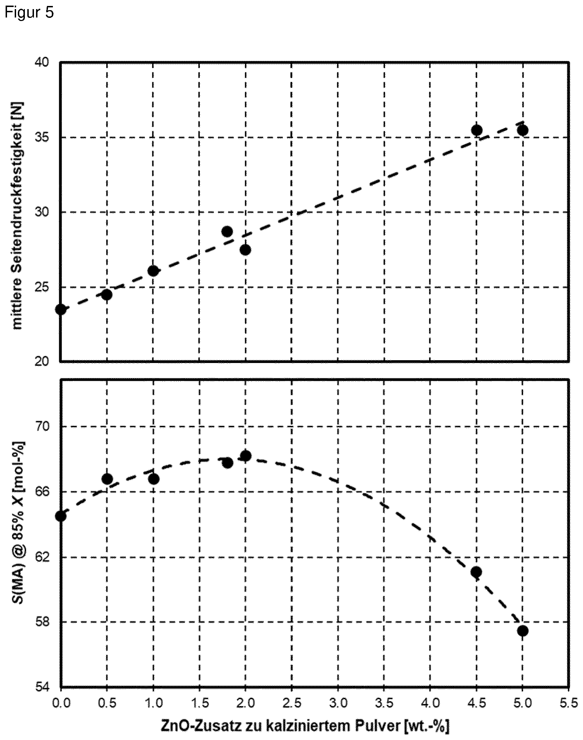

Die in Tabelle 1 und in Figur 5 dargestellten Ergebnisse zeigen, dass durch die Zugabe von ZnO bzw. ZnO/MgO nach dem Kalzinierungsschritt, bei einer Messung direkt nach der Tablettierung keine systematische Erhöhung der Seitendruckfestigkeit (SDF) zu beobachten ist. Überraschenderweise ist dies jedoch nach dem darauffolgenden Aktivierungsschritt der VPO-Katalysator-Tabletten der Fall. Hier zeigt sich eine systematische und sehr deutliche Erhöhung der Seitendruckfestigkeit der Partikel mit Zusatz von ZnO bzw. ZnO/MgO. Tabelle 1 und Figur 5 zeigen auch, dass unter den gewählten katalytischen Bedingungen (Umsatz 85 %) die Zugabe von ZnO bzw. ZnO/MgO zu einer Erhöhung der Selektivität führt.

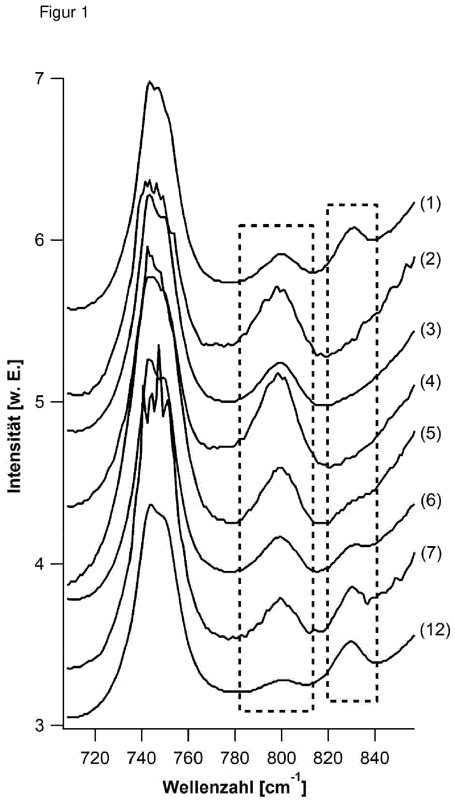

Der erfindungsgemäße VPO-Katalysator charakterisiert sich darüber hinaus durch infrarotspektroskopische Merkmale. Übliche nicht erfindungsgemäße VPO-Katalysatoren ohne ZnO weisen im IR-Spektrum ein Maximum bei 790 cm⁻¹ bis 810 cm⁻¹ (erste Absorptionsbande) und ein zweites Maximum zwischen 820 cm-1 bis 840 cm-1 (zweite Absorptionsbande) auf, wobei hierbei die zweite Absorptionsbande stärker ist als die erste Absorptionsbande (Figur 1, (1)). Es wurde festgestellt, dass mit der Zugabe von ZnO bzw. ZnO/MgO gleichzeitig eine Zunahme der Stärke der Bande bei 790 cm⁻¹ bis 810 cm⁻¹ (erste Absorptionsbande) und eine Abnahme der Stärke des Maximums zwischen 820 cm⁻¹ bis 840 cm⁻¹ (zweite Absorptionsbande) auftritt, sodass das die erste Absorptionsbande bei den erfindungsgemäßen Proben stärker ist als die Stärke der zweiten Absorptionsbande oder die Stärke der zweiten Absorptionsbande sogar so erniedrigt wird, dass sie nicht mehr detektierbar ist (Figur 1, (2) - (7)). Im letzteren Fall zeichnen sich die erfindungsgemäßen VPO-Katalysatoren dadurch aus, dass sie nur noch die erste Absorptionsbande aufweisen.

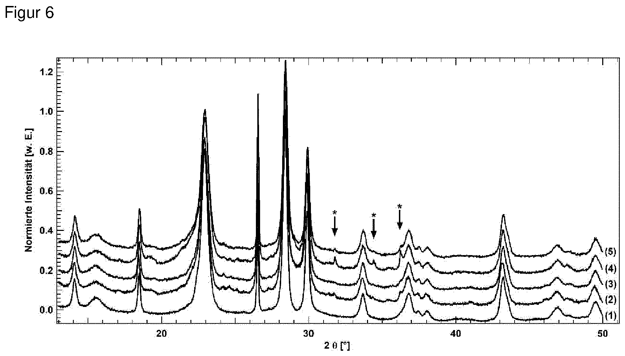

Es zeigt sich auch, dass die erfindungsgemäßen VPO-Katalysatoren folgende, durch XRD-Diffraktometrie, beobachtbare Merkmale aufweisen (Figur 6):
31,7 bis 31,9° (scharfer Reflex, ZnO)
34,3 bis 34,5° (scharfer Reflex, ZnO)
36,2 bis 36,4° (scharfer Reflex, ZnO)

Diese Merkmale werden mit steigendem ZnO-Gehalt intensiver und stimmen gut mit ZnO (in Wurzitstruktur bzw. als Zinkit) überein.

### Vergleichsbeispiel 8, Beispiele 9, 10

Um den Zusammenhang zwischen dem Vorliegen der stabilisierenden ZnO-Phase und dem Zugabezeitpunkt des Bindemittels zu erfassen, wurden nach verschiedenen Zugabezeitpunkten XRD-Diffraktogramme aufgenommen. Während bei den Beispielen 2 bis 7 ZnO immer nach der Kalzinierung und vor der Tablettierung zugegeben wurde, erfolgte die Zugabe des ZnO zum Zeitpunkt der Herstellung der Reaktionsmischung während Verfahrensschritt a), d.h. zusammen mit der Zugabe des V₂O₅ (Vergleichsbeispiel 8), unmittelbar nach der Filtration nach Verfahrensschritt c) (Beispiel 9), während Verfahrensschritt d), d.h. nach der Vakuumtrocknung und vor der Kalzinierung (Beispiel 10). Es wurden hierbei jeweils 5,2 g ZnO zugegeben, sodass die fertigen Katalysatoren nominal jeweils 2 Gew.-% ZnO enthielten.

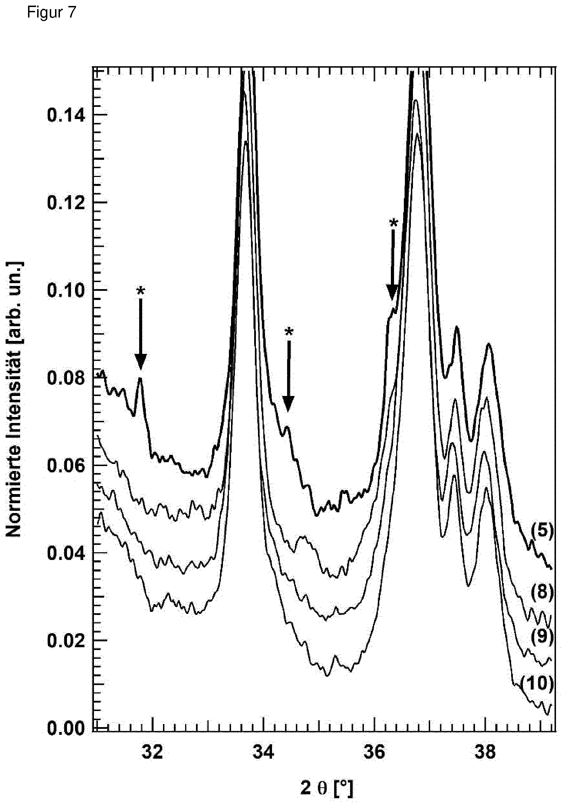

Figur 7, zeigt, dass die bei der Zugabe von ZnO nach der Kalzinierung und vor der Tablettierung beobachteten XRD-Reflexe, insbesondere bei 31,7 bis 31,9°, 34,3 bis 34,5°, 36,2 bis 36,4° nicht sichtbar sind, wenn das ZnO zu Beginn des Refluxschritts zugegeben wird. (Figur 7, Diffraktogramm (8).

### Vergleichsbeispiele 11 und 12

Die VPO-Katalysatoren gemäß den Vergleichsbeispielen 11 und 12 wurden analog wie der VPO-Katalysator nach Beispiel 1 hergestellt, wobei die aktivierten Tabletten mit Zn(OAc)₂-Lösung imprägniert wurden. Hierbei wurden zwei unterschiedliche Konzentrationen verwendet, um ein Zn/V Verhältnis von 0,008 (= 0,29 Gew.-% Zn bezogen auf das Gesamtkatalysatorgewicht) bzw. 0,016 (entspricht 0,58 Gew.-% bezogen auf das Gesamtkatalysatorgewicht), zu erhalten. Nach der Imprägnierung wurden die Katalysatoren erst für 18 h bei 100°C und anschließend noch für 20 min. bei 350°C in Luft getrocknet.

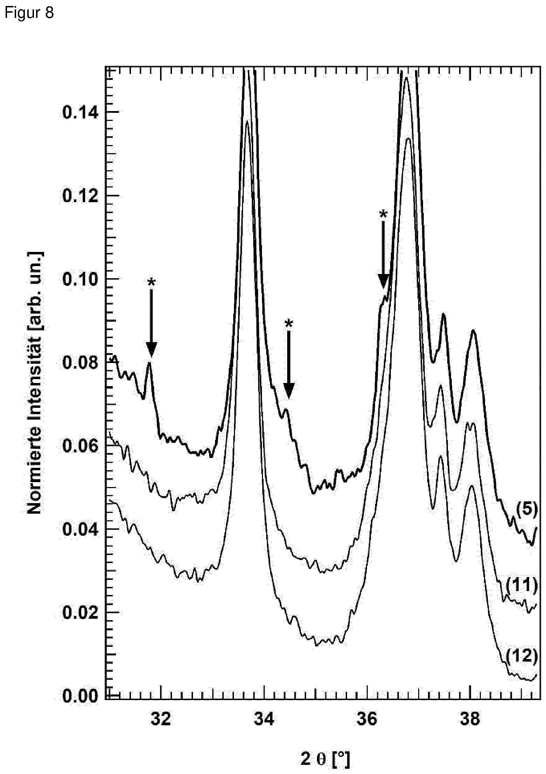

Figur 8 zeigt die XRD-Diffraktogramme der VPO-Katalysatoren gemäß dem Vergleichsbeispielen 11 und 12 (Diffraktogramme (11) und (12)) im Vergleich zu dem die gemäß der Erfindung hergestellten VPO-Katalysator nach Beispiel 5 (Diffraktogramm (5)). Es ist ersichtlich, dass durch die Zugabe der ZnO-Verbindung zu den aktivierten Tabletten keine Reflexe insbesondere bei 31,7 bis 31,9°, 34,3 bis 34,5°, 36,2 bis 36,4° zeigen.

### Vergleichsbeispiele 13 und 14

Der VPO-Katalysator gemäß Vergleichsbeispiel 13 wurden analog wie der VPO-Katalysator nach Beispiel 2 hergestellt, wobei zum kalzinierten Pulver 10 Gew.-% Bindemittel vom Typ SECAR 71 und 4 Gew.-% Graphit zugegeben wurden.

Die Stabilität eines VPO-Katalysators kann sehr gut durch Messung der Stabilität im Hinblick auf das Nebenprodukt Wasser erfasst werden, denn während der Reaktion ist, bedingt durch die Oxidationsreaktion, ein erheblicher Anteil Wasserdampf anwesend, der den Katalysator schädigen kann. Die Stabilität im Hinblick auf das Nebenprodukt Wasser, des erfindungsgemäßen VPO-Katalysators nach Beispiel 4 im Vergleich zu einem mit herkömmlichen Binder hergestellten VPO-Katalysators nach Vergleichsbeispiel 13, wurde daher getestet. Hierzu wurde die Selektivität der unbehandelten Proben getestet, anschließend wurden beide Proben mit Wasser imprägniert und der Test der Selektivität wiederholt (Vergleichsbeispiel 14). Es zeigt sich, dass der erfindungsgemäße Katalysator durch die Wasserimprägnierung innerhalb der Messtoleranz keinen Selektivitätsverlust erleidet.

### Beispiel 15 und 16

Die VPO-Katalysatoren gemäß den Beispielen 15 und 16 wurden analog wie der VPO-Katalysator nach Beispiel 1 dargestellt, aber ohne Zugabe von Ammoniumdimolybdat, so dass der erhaltene VPO-Katalysator frei von Mo war.

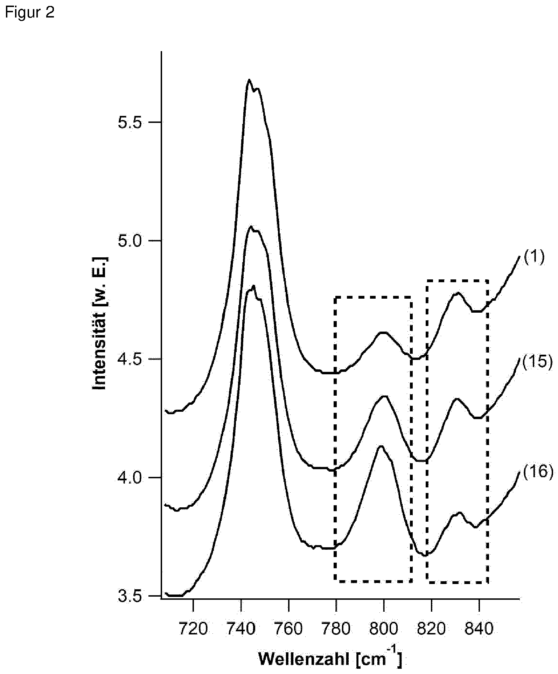

Außerdem wurde nach der Kalzinierung und vor der Tablettierung 1 Gew.-% (Beispiel 15), bzw. 2 Gew.-% ZnO (Beispiel 16), bezogen auf das Gesamtgewicht des kalzinierten Pulvers, zum kalzinierten Pulver zugegeben. Wie Tabelle 1 zeigt, wird auch bei den Mo-freien VPO-Katalysatoren eine Erhöhung der katalytischen Selektivität in Kombination mit einer Erhöhung der Seitendruckfestigkeit erzielt. Die in Figur 2 gezeigten, in Transmission durchgeführte IR-Spektren zeigen zudem, dass bei diesen beiden erfindungsgemäßen Proben, die Intensität der Bande bei 790 cm⁻¹ bis 810 cm⁻¹ (erste Absorptionsbande), stärker ist als das Maximum zwischen 820 cm⁻¹ bis 840 cm⁻¹.

### Beispiele 17 und 18 (Vergleich)

### Verwendete Apparatur

Es wurde dieselbe Apparatur wie in Beispiel 1 verwendet.

### Herstellen der Katalysatorvorstufe

Als erstes werden 150 g V₂O₅ im Vierhalskolben vorgelegt. Hierzu werden 300 mL Benzylalkohol und 1200 mL Isobutanol gegeben. Nach Inertisieren mit N₂ wird die Suspension unter Rühren 10h auf Rückfluss geheizt. Nach Abkühlen auf maximal 40°C unter Rühren werden 7,51 g Ammoniumdimolybdat, 6,60 g Eisen(III)nitratnonahydrat, 2,55g Cer(III)nitrathexahydrat und 4,5 g Niobammoniumoxalat zugegeben. Nach Zugabe von 138 mL Phosphorsäure (85% ig) unter Rühren innerhalb von 15 min. und Inertisieren mit N₂ wird die Suspension unter Rühren nochmals für 24 h auf Rückfluss geheizt.

### Filtrieren:

Nach Abkühlen der Suspension, die das Katalysatorvorprodukt enthält, wird diese aus dem Vierhalskolben in eine Filternutsche übertragen und die Flüssigkeit abgesaugt. Der feuchte Filterkuchen wird einmal mit Ethanol (100%) gewaschen und wieder in der Filternutsche die Flüssigkeit abgesaugt. Anschließend erfolgt die gleiche Prozedur nochmal mit bidest. Wasser.

### Trocknung / Kalzinierung:

Der gewaschene Filterkuchen wird in den Verdampferkolben eines Rotationsverdampfers gefüllt. Unter Wasserstrahlvakuum wird der Filterkuchen bei 120 °C über Nacht getrocknet. Das so getrocknete Pulver wird in einem geeigneten Kalziniertopf in einen Ofen gestellt und in einer N₂-Atmosphäre bei Temperaturen von 200 bis 300 °C für 9 h kalziniert.

### Kompaktierung/Tablettierung:

Vor der Kompaktierung/Tablettierung werden dem kalzinierten pulverförmigen Katalysatorvorprodukt zuerst 2,1 Gew.-% Zn₃(PO₄)₂ und dann 4 Gew.-% Graphit zugegeben und mit Hilfe eines Rhönradmischers homogen durchmischt. Dieses Pulver wird mit einem Walzenkompaktor mit einem Anpressdruck von 190 bar, einer Spaltbreite 0,60 mm und einer Walzengeschwindigkeit von 7 U/min, zu Platten kompaktiert und durch ein 1 mm Sieb granuliert.

Das Granulat wird mit einer Rundläufertablettenpresse zu der gewünschten Tablettenform, mit entsprechender Höhe, z.B. 5,6 x 5,6 x 2,3 mm, und Seitendruckfestigkeit, gepresst.

### Temperaturbehandlung

Die folgenden Temperaturbehandlung wird in einer in einem programmierbaren Ofen eingebauten Retorte unter kontrollierten Bedingungen durchgeführt. Die kalzinierten Tabletten werden gleichmäßig in die Retorte eingefüllt und diese wird dicht verschlossen. Danach wird der Katalysator in einem Luftstrom auf 120°C für 18 h behandelt.

Der VPO-Katalysator gemäß Beispiel 18 wurde analog wie der VPO-Katalysator nach Beispiel 1 dargestellt, wobei nach der Kalzinierung und vor der Tablettierung 2,1 Gew.-% Zn₃(PO₄)₂ bezogen auf das Gesamtgewicht des kalzinierten Pulvers, zum kalzinierten Pulver zugegeben.

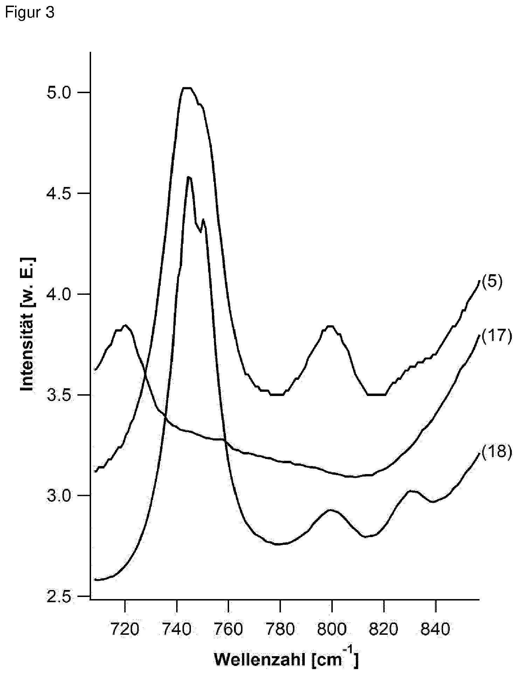

Wie in Tabelle 1 gezeigt wird, zeigen die nicht erfindungsgemäßen Proben nach den Beispielen 17 und 18 eine deutlich niedrigere Selektivität im Vergleich zu den erfindungsgemäßen Proben. Gleichzeitig zeigt sich bei den in Transmission durchgeführte IR Spektren in Figur 3, dass bei diesen beiden nicht-erfindungsgemäßen Proben, die Intensität der Bande bei 790 cm⁻¹ bis 810 cm⁻¹ (erste Absorptionsbande), nicht stärker ist als das Maximum zwischen 820 cm⁻¹ bis 840 cm⁻¹ (Beispiel 17) oder beide Banden nicht vorhanden sind (Beispiel 18).

### Ergebnisse

**Tabelle 1:**

| **Beispiel** | **Bindemittel** | **Menge Bindemittel [Gew.%]** | **Seitendruckfestigkeit vor Aktivierung [N]** | **Seitendruckfestigkeit nach Aktivierung [N]** | **IR, XRD Fig.** | **Selektivität 85% (MA) [%]** |
|---|---|---|---|---|---|---|
| 1 (Vergleich) | - | 0,0 | 23,4 | 23,5 | 1,6 | 64,5 |
| 2 (Erfindung) | 9ZnO / 1MgO* | 5,0 | 24,1 | 35,5 | 1,6 | 61,1 |
| 3 (Erfindung) | 9ZnO / 1MgO* | 2,0 | 26,1 | 28,7 | 1,6 | 67,8 |
| 4 (Erfindung) | ZnO | 5,0 | 21,1 | 35,5 | 1,6 | 58,5 |
| 5 (Erfindung) | ZnO | 2,0 | 20,9 | 27,5 | 1,6,7, 8 | 68,2 |
| 6 (Erfindung) | ZnO | 1,0 | 20,2 | 26,1 | 1 | 66,8 |
| 7 (Erfindung) | ZnO | 0,5 | 22,8 | 24,6 | 1 | 66,8 |
| 8 (Vergleich) | ZnO | 2,0 | - | - | 7 | - |
| 9 (Erfindung) | ZnO | 2,0 | - | - | 7 | - |
| 10 (Erfindung) | ZnO | 2,0 | - | - | 7 | - |
| 11 (Vergleich) | Zn(OAc)₂ | 0,3 | - | - | 8 | 64,8 |
| 12 (Vergleich) | Zn(OAc)₂ | 0,6 | - | - | 8 | 59,9 |
| 13 (Vergleich) | SECAR | 10 | - | - | - | 64,6 |
| 14 (Vergleich) | SECAR | 10 | - | - | - | 58,0 |
| 15 (Erfindung) | ZnO | 1,0 | 17,6 | 23,3 | 2 | 66,9 |
| 16 (Erfindung) | ZnO | 2,0 | 15,7 | 27,5 | 2 | - |
| 17 (Vergleich) | Zn₃(PO₄)₂ | 2,1 | 19,3 | 13,9 | 3 | 65,3 |
| 18 (Vergleich) | Zn₃(PO₄)₂ | 2,1 | 24,0 | 14,3 | 3 | 62,5 |

### Methoden

### Infrarotspektroskopie (IR)

Die Katalysatoren wurden mittels Infrarotspektroskopie (IR) in Transmission charakterisiert. Hierbei wird wellenlängenabhängig die Intensität der Infrarotstrahlung einer Quelle (I₀) nach Durchgang durch eine Probe (I) bestimmt. Durch Absorption der IR-Strahlung und damit Anregung von Schwingungsmoden im untersuchten Material kommt es wellenlängenabhängig zur Abschwächung der Infrarotstrahlung. Aufgetragen wird für das Spektrum die Absorption, berechnet über log(I₀/I), gegen die Wellenzahl.

Die Messungen wurden an einem Nicolet Nexus 470 FTIR Spektrometer mit mittels flüssig Stickstoff gekühltem Quecksilber-Cadmium-Tellur (MCT) Detektor, einer "IR Source Everglo" als IR-Quelle und einem HeNe-Laser zur Frequenzkalibrierung durchgeführt. Für die Probenherstellung wurden Tabletten der jeweiligen Katalysatoren fein gemörsert und mittels Handstempelpresse wurde mit ca. 10 mg Material ein dünner Pressling von 11 mm Durchmesser erzeugt. Dieser wurde in einer Vakuumkammer mit KBr-Fenstern montiert und im Strahlengang platziert. Anschließend wurde die Probe für 2h bei 200°C und einem Druck von 10⁻⁴ mbar vorbehandelt, um adsorbiertes Wasser zu entfernen. Für die Messungen wurde die Vakuumkammer mit der Probe mittels flüssig Stickstoff auf 77 K abgekühlt und bei einem Druck von 10⁻⁴ mbar mit einer Schrittweite von 2 cm⁻¹, einer Apertur von 72 und einer optical velocity von 0.64 das Spektrum aufgezeichnet.

### Bestimmung des IR-Absorptionsmaximums

Die Stärke der beiden Absorptions-Banden mit einem Maximum jeweils im Bereich zwischen 790 cm⁻¹ bis 810 cm⁻¹ und 820 cm⁻¹ bis 840 cm⁻¹ entspricht der Stärke der Absorption im Bereich des Maximums der Bande im Vergleich zum spektralen Untergrund. Für die Bestimmung der Stärke der Absorption im Bereich des Maximums wurde jeweils eine erste Gerade als linearer Untergrund so gesetzt, dass diese das Spektrum, d.h. den Untergrund des Spektrums, unmittelbar (d. h. innerhalb des Frequenzbereichs bis zu einer benachbarten Absorptionsbande) links und rechts unterhalb der betreffenden Bande tangential berührt. Anschließend wird vom Maximum der Bande (S0) eine Linie senkrecht nach unten geführt, d.h. in Richtung gleichbleibender Wellenlänge und abnehmender Absorption, und der Schnittpunkt dieser Linie mit dem linearen Untergrund (S1) bestimmt. Die Stärke der Absorptionsbande ist definiert als die Länge der Strecke S0 zu S1.

### Test der Bruchfestigkeit

Zur Messung der Bruchfestigkeit der Formkörper wurde ein Zwick Z0.5 Gerät verwendet, um die für den Bruch benötigte Kraft zu bestimmen. Die Messungen wurden gemäß der Norm ASTM D4179 durchgeführt. Zur Trocknung der Formkörper vor der Messung wurden diese für mindestens 3 h bei 100°C im Trockenschrank gelagert und die anschließende Messung der Bruchfestigkeit innerhalb von höchstens 1h nach Ende der Trocknung durchgeführt. Betrieben wurde das Gerät gemäß der Norm ASTM D4179 bei einer konstanten Kraftrate von 20,0 N/s. Es wurden für jedes Beispiel nacheinander 100 Tabletten jeweils einzeln mit der Zylinderachse parallel zur Messbackenoberfläche platziert (in der Norm ASTM D4179 der "RADIAL CRUSH") und vermessen. Aus den 100 Einzelwerten der jeweils für den Bruch des Formkörpers erforderlichen Kraft wurde dann der Mittelwert bestimmt, der der durchschnittlichen Bruchkraft des Formkörpers entspricht. Sämtliche Angaben zur Seitendruckfestigkeit in dieser Anmeldung beziehen sich auf Seitendruckfestigkeiten, die durch die hier beschriebene Methode erhalten wurden.

### Pulver-Röntgen-Diffraktometrie (XRD)

Die Katalysatoren wurden mittels Röntgenpulverdiffraktometrie (X-ray diffraction; XRD) charakterisiert. Hierbei wird Röntgenstrahlung an den kristallinen Bereichen der Probe in verschiedenen Beugungswinkeln gebeugt. Der Beugungswinkel wird in 2θ gemessen bzw. aufgetragen. Dabei kommt es je nach vorhandener Phase zu charakteristischen Reflexen in Abhängigkeit des Beugungswinkels. Anhand dieser Beugungsmuster kann mithilfe einer Datenbank eine Zuordnung des Beugungsmusters zu vorhandenen Phasen erfolgen.

Die Messungen wurden an einem D4 Endeavor der Firma Bruker AXS mit Cu-Ka-Strahlung und einem LYNXEYE-Detektor durchgeführt. Die Diffraktrogramme wurden in einem Winkelbereich 2θ von 5 bis 50 ° mit einer Schrittweite von 0,02° bei einer Aufnahmezeit von 1,5 s pro Schritt mit einer fixen Divergenzblende von 0,3° aufgenommen. Zur Messung wurden die Proben fein gemörsert und in einem Probenhalter verpresst. Im Apparat wurde die Variation des Beugungswinkels durch Kippen der Probe ermöglicht. Sämtliche Angaben zu XRD-Reflexen in diese Anmeldung beziehen sich auf mit dieser Methodik erhaltene XRD-Reflexe.

Zur besseren Vergleichbarkeit der Diffraktogramme wurden diese jeweils normiert. Hierzu wird für jedes Diffraktogramm zuerst der Datenpunkt mit dem kleinsten Intensitätswert gesucht. Dieser Wert wird von allen Intensitätswerten des jeweiligen Diffraktogramms abgezogen. Anschließend wird die maximale Intensität des (024) Reflexes (liegt bei einem 2θ Wert von 28.4-28.5°) im jeweiligen Diffraktogramm ermittelt. Durch diesen Wert werden alle Intensitätswerte des jeweiligen Diffraktogramms dividiert.

### Katalytische Testreaktion

Zur Ermittlung der Katalysatorperformance wurden alle Katalysatoren nach erfolgter vollständiger Präparation (Reflux, Filtration, Vakuumtrocknung, Kalzinierung, Kompaktierung, Tablettierung, Aktivierung) in einem "Bench-Scale"-Reaktor bei 1,5 mol-% Butan in Luft in einem verdünnten Katalysatorbett (1:9 Mischung Katalysator: keramische Inertringe) hinsichtlich ihrer katalytischen Eigenschaften getestet. Die Selektivität zu Maleinsäureanhydrid (MA) wurde bei einer Reaktionstemperatur von 410°C (Salzbad) über eine Variation der massenbezogenen GHSV im Bereich 1300 bis 5500 l/kg/h durch Interpolation auf einen Umsatz von 85 % ermittelt.

## Patentansprüche

1. VPO-Katalysator in Form eines Formkörpers zur Oxidation von Kohlenwasserstoffen mit molekularem Sauerstoff, insbesondere zur Oxidation von Butan zu Maleinsäureanhydrid mit molekularem Sauerstoff, wobei der VPO-Katalysator zwischen 0,05 Gew.-% bis 7,0 Gew.-% Zn enthält, welches teilweise als ZnO vorliegt und bei in Transmission durchgeführter Infrarotspektroskopie eine erste Absorptionsbande mit einem Maximum zwischen 790 cm⁻¹ bis 810 cm⁻¹ und eventuell eine zweite Absorptionsbande mit einem Maximum zwischen 820 cm⁻¹ bis 840 cm⁻¹ aufweist, **dadurch gekennzeichnet, dass** nur die erste Absorptionsbande vorhanden ist oder die Stärke der ersten Absorptionsbande größer ist als die Stärke der zweiten Absorptionsbande.

2. VPO-Katalysator nach Anspruch 1, **dadurch gekennzeichnet, dass** die Stärke der Absorptionsbande bestimmt wird, indem jeweils eine erste Gerade so gesetzt wird, dass diese das Spektrum unmittelbar links und rechts unterhalb der betreffenden Bande tangential berührt, anschließend eine zweite Gerade ausgehend vom Maximum der Bande in Richtung gleichbleibender Wellenlänge und abnehmender Absorption geführt wird und der Schnittpunkt der beiden Geraden bestimmt wird, wobei die Stärke der Absorptionsbande definiert ist als die Länge zwischen Maximum und Schnittpunkt.

3. VPO-Katalysator nach Anspruch 1 oder 2, **dadurch gekennzeichnet, dass** das Verhältnis zwischen der Stärke der ersten Absorptionsbande und der Stärke der zweiten Absorptionsbande über 1,1 vorzugsweise über 2 liegt.

4. VPO-Katalysator nach einem der vorhergehenden Ansprüche, **dadurch gekennzeichnet, dass** der VPO-Katalysator einen Mo-Gehalt zwischen 0,1 Gew.-% und 1 Gew.-%, bevorzugter Weise zwischen 0,4 Gew.-% und 0,7 Gew.-% Mo, jeweils bezogen auf das Gesamtgewicht des VPO-Katalysators, enthält.

5. VPO-Katalysator nach einem der vorhergehenden Ansprüche, **dadurch gekennzeichnet, dass** der VPO-Katalysator zwischen 0,1 Gew.-% und 6 Gew.-%, bevorzugterweise zwischen 0,2 Gew.-% und 4 Gew.-% Zn enthält, stärker bevorzugt zwischen 0,5 Gew.-% und 3,5 Gew.-%, bezogen auf das Gesamtgewicht des VPO-Katalysators.

6. VPO-Katalysator nach einem der vorhergehenden Ansprüche, **dadurch gekennzeichnet, dass** der VPO-Katalysator folgende elementare Zusammensetzung aufweist:
- 0,5 Gew.-% bis 7 Gew.-% Zn,
- 0 Gew.-% bis 0,7 Gew.-% Mo,
- 26 Gew.-% bis 31 Gew.-% V,
- 17 Gew.-% bis 21 Gew.-% P,
- 3 Gew.-% bis 5 Gew.-% C,
im übrigen Sauerstoff, jeweils bezogen auf das Gesamtgewicht des VPO-Katalysators.

7. VPO-Katalysator nach einem der vorhergehenden Ansprüche, **dadurch gekennzeichnet, dass** der Formkörper eine Seitendruckfestigkeit von mehr als 25 N, vorzugsweise zwischen 25 N bis 200 N aufweist, wobei die Seitendruckfestigkeit mit einem Zwick Z0.5 Gerät unter Verwendung der Norm ASTM D4179 mit einer konstanten Kraftrate von 20,0 N/s gemessen wurde, wobei 100 Tabletten jeweils einzeln mit der Zylinderachse parallel zur Messbackenoberfläche platziert und vermessen wurden, um eine durchschnittliche Bruchkraft zu bestimmen, die die Seitendruckfestigkeit darstellt.

8. VPO-Katalysator nach einem der vorhergehenden Ansprüche, **dadurch gekennzeichnet, dass** der VPO-Katalysator, wenn er mit Pulver-Röntgendiffraktometrie untersucht wird, Reflexe bei 31,7 ° bis 31,9 °, 34,3 ° bis 34,5 ° und 36,2 ° bis 36,4 ° aufweist, bei einer Messung mit einem D4 Endeavor der Firma Bruker AXS mit Cu-Ka-Strahlung und einem LYNXEYE-Detektor, wobei die Diffraktrogramme in einem Winkelbereich 2θ von 5 ° bis 50 ° mit einer Schrittweite von 0,02 ° bei einer Aufnahmezeit von 1,5 s pro Schritt mit einer fixen Divergenzblende von 0,3 ° aufgenommen werden.

9. VPO-Katalysator nach einem der vorhergehenden Ansprüche, **dadurch gekennzeichnet, dass** der Formkörper eine Doppelalphaform aufweist, mit einer Höhe von 3 mm bis 8 mm, einer Länge von 5 mm bis 9 mm, einer Breite von 4 mm bis 8 mm und einem Lochinnendurchmesser von 1 mm bis 4 mm.

10. VPO-Katalysator nach einem Ansprüche 1 bis 8, **dadurch gekennzeichnet, dass** der Formkörper eine zylindrische Form aufweist, mit einer Höhe von 3 mm bis 8 mm, einer im wesentlichen runden Grundfläche mit einem Durchmesser 3 mm bis 8 mm und einer mittleren axialen Öffnung mit einem Durchmesser von 1 mm bis 3 mm.

11. Verfahren zur Herstellung eines VPO-Katalysators nach einem der Ansprüche 1 bis 10, umfassend die Schritte:
a) Herstellen einer Katalysatorvorstufe enthaltend Vanadylhydrogenphosphat,
b) Formen der Katalysatorvorstufe zu Formkörpern,
c) Aktivieren der Formkörper, um eine VPO-Phase auszubilden,
**dadurch gekennzeichnet, dass** nach Schritt a) ZnO mit der Katalysatorvorstufe vermischt wird.

12. Verfahren zur Herstellung eines VPO-Katalysators nach Anspruch 11, **dadurch gekennzeichnet, dass** die Herstellung der Katalysatorvorstufe enthaltend Vanadylhydrogenphosphat in Schritt a), durch die Reduktion einer V(V)-Verbindung in Gegenwart einer P(V)-Verbindung durch ein organisches Reduktionsmittel während eines Refluxschritts bei Normaldruck in einem organischen Lösungsmittel erfolgt.

13. Verfahren zur Herstellung eines VPO-Katalysators nach Anspruch 11 oder 12, **dadurch gekennzeichnet, dass** die in Schritt a) erhaltene Katalysatorvorstufe in einem Schritt a₁) filtriert wird und in einem Schritt a₂) bei einer Temperatur von maximal 300 °C getrocknet und / oder kalziniert wird.

14. Verfahren zur Herstellung eines VPO-Katalysators nach Anspruch 12 oder 13, **dadurch gekennzeichnet, dass** Schritt a₂) in zwei Schritten erfolgt, wobei in einem ersten Schritt in Vakuum bei einer Temperatur im Bereich von 90 °C bis 140 °C während eines Zeitraums von 1 h bis 24 h getrocknet wird und in einem zweiten Schritt in Stickstoff bei einer Temperatur im Bereich von 230 °C und 290 °C während eines Zeitraums von 1 h bis 24 h kalziniert wird.

15. Verfahren zur Herstellung eines VPO-Katalysators nach Anspruch 11 bis 14, **dadurch gekennzeichnet, dass** der Aktivierungsschritt in einer Gasmischung bestehend aus Luft, Inertgas und Wasserdampf, bei einer Temperatur im Bereich von 300 °C bis 500 °C, vorzugsweise im Bereich von 350 °C bis 450 °C, während eines Zeitraums von 1 h bis 24 h durchgeführt wird.

16. Verfahren zur Herstellung eines VPO-Katalysators nach einem der Ansprüche 11 bis 15, **dadurch gekennzeichnet, dass** nach Schritt a) die Katalysatorvorstufe zusätzlich mit einer Mg-Verbindung, vorzugweise MgO vermischt wird.

17. Verwendung von ZnO zur Stabilisierung eines VPO-Katalysators in Partikelform.

18. Verwendung nach Anspruch 17, bei der der VPO-Katalysator in Form eines Formkörpers nach dem Verfahren gemäß Anspruch 11 hergestellt wird.

19. Verwendung von ZnO als Bindemittel für einen VPO-Katalysator in Form eines Formkörpers.

20. Verfahren zur Herstellung von Maleinsäureanhydrid durch katalytische Oxidation von n-Butan, wobei ein Eduktgas umfassend Sauerstoff und n-Butan durch eine Reaktorröhre geleitet wird, in der sich eine Schüttung von VPO-Katalysatoren nach einem der Ansprüche 1 bis 9 befindet.

21. Verfahren nach Anspruch 20, **dadurch gekennzeichnet, dass** die Schüttung der VPO-Katalysatoren in der Reaktorröhre bei einer Temperatur zwischen 300 °C und 420 °C vorliegt.

22. Verfahren nach einem der Ansprüche 20 oder 21, **dadurch gekennzeichnet, dass** das Eduktgas zwischen 0,2 bis 10 Vol.-% n-Butan und zwischen 5 und 50 Vol.-% Sauerstoff enthält und mit einer Raumzeitgeschwindigkeit von 1100 h⁻¹ bis 2500 h⁻¹, vorzugsweise 1300 h⁻¹ bis 1600 h⁻¹ durch die Reaktorröhre geleitet wird.
